# EUROPÄISCHE PATENTSCHRIFT

(11) **EP 3 390 713 B1**
(45) Veröffentlichungstag und Bekanntmachung des Hinweises auf die Patenterteilung: **18.09.2019**
(21) Anmeldenummer: 16820196.0
(22) Anmeldetag: 14.12.2016
(51) Int. Cl.: D21F 7/06, D21G 9/00, G01N 33/34, G01N 21/89

(54) **VERFAHREN UND VORRICHTUNG ZUR BESTIMMUNG DES FLÄCHENGEWICHTS EINER FASERSTOFFBAHN**
METHOD AND APPARATUS FOR ESTABLISHING THE WEIGHT PER UNIT AREA OF A FIBROUS WEB
PROCÉDÉ ET DISPOSITIF DE DÉTERMINATION DE LA MASSE SURFACIQUE D'UNE BANDE DE MATIÈRE FIBREUSE

(30) Priorität: 18.12.2015 DE 102015225962
(43) Veröffentlichungstag der Anmeldung: 24.10.2018
(73) Patentinhaber: Voith Patent GmbH, 89522 Heidenheim (DE)
(72) Erfinder: HARDT, Niels, 89542 Herbrechtingen (DE); BICKERT, Martin, 56179 Vallendar (DE); VÄTH, Eduard, 56357 Geisig (DE); GROSS, Michael, 56566 Neuwied (DE)
(86) Internationale Anmeldenummer: PCT/EP2016/080871
(87) Internationale Veröffentlichungsnummer: WO 2017/102774

(56) Entgegenhaltungen:
- EP-A1- 1 557 659
- EP-A2- 0 311 507
- DE-A1-102011 082 845
- DE-U1-202007 004 135

## Beschreibung

Die Erfindung betrifft ein Verfahren sowie eine Vorrichtung zur Bestimmung des Flächengewichts einer aus einer Stoffsuspension hergestellten Faserstoffbahn, insbesondere Papier-, Karton- oder Tissuebahn.

Bei der industriellen Herstellung von Faserstoffbahnen, insbesondere Papier-, Karton- oder Tissuebahnen, wird bei der bisher angewandten Online-Flächengewichtsmesstechnik für eine möglichst genaue Ermittlung des Flächengewichtswertes der Faserstoffbahn meistens auf radioaktive Messtechniken zurückgegriffen. Es ist auch bereits bekannt, auf der Basis infrarotoptischer Absorptionsmesstechniken das Faser- oder Zellulosegewicht und den Wassergehalt einer jeweiligen Faserstoffbahn zu bestimmen.

Papier besteht im Wesentlichen aus Zellulose, Wasser und Füllstoffen (Asche). Aus dem Stand der Technik, insbesondere den Schriften DE 10 2011 082845, EP 1 557 659, DE 20 2007 004135 und EP 0 311 502 sind hierfür verschiedene Messsysteme bekannt.

Dabei erfassen die bisherigen infrarotoptischen Messtechniken nur den Zellulose- und Wasseranteil bzw. nur das Faser- oder Zellulosegewicht und den Wassergehalt.

Bei Füllstoffen handelt es sich meistens um anorganische Stoffe entweder mineralischer oder chemischer gefällter Herkunft. Sie werden in der Regel zugegeben, um das Papier in seinen Eigenschaften zu verändern oder um Faserstoff zu sparen. Das Einbringen von Füllstoffen in das Fasergefüge dient insbesondere dazu, Lücken zwischen den Fasern zu schließen, d.h. zu füllen, bestimmte Papiereigenschaften wie beispielsweise einen höheren Weißgrad, eine höhere Opazität, eine geschlossene Oberfläche und Güte zu erzielen, die Saugfähigkeit zu beeinflussen sowie die Bedruckbarkeit zu verbessern.

Da der Füllstoffanteil (Asche) durch die infrarottechnischen Messtechniken nicht erfasst wird, wird bisher bei der Verwendung solcher infrarotoptischer Messtechniken bei konstantem bekanntem Füllstoffanteil vom Fasergewicht auf das Flächengewicht entsprechend hochgerechnet. Prozessbedingt treten nun aber häufig Situationen auf, in denen es zu Schwankungen des Füllstoffgehalts kommt, was insbesondere bei der Herstellung von Tissuepapieren der Fall ist. Dabei können die Schwankungen des Füllstoffgehalts im Bereich von 0 bis 10% liegen. Da die jeweils auftretenden Schwankungen unbekannt sind, können sie die Bestimmung des Flächengewichts aus dem Fasergewicht verfälschen und bringen somit eine geringere Genauigkeit der Flächengewichtsbestimmung mit sich. Entsprechend sind auch die Schwankungen des Flächengewichts insbesondere bei Tissuepapier sehr stark. Es kann in einem Bereich von 8 bis 60 g/m² variieren.

Der Erfindung liegt die Aufgabe zugrunde, ein Verfahren sowie eine Vorrichtung der eingangs genannten Art anzugeben, mit denen ein genauere auf nicht-radiometrischen Messprinzipien basierende Bestimmung des Flächengewichts einer Faserstoffbahn wie insbesondere einer Papier-, Karton- oder Tissuebahn gewährleistet ist.

Erfindungsgemäß wird die Aufgabe durch ein Verfahren mit den Merkmalen des Anspruchs 1 bzw. eine Vorrichtung mit den Merkmalen des Anspruchs 7 gelöst.

Bevorzugte Ausgestaltungen des erfindungsgemäßen Verfahrens sowie bevorzugte Ausführungsformen der erfindungsgemäßen Vorrichtung ergeben sich aus den Unteransprüchen, der vorliegenden Beschreibung sowie der Zeichnung.

Das erfindungsgemäße Verfahren zur Bestimmung des Flächengewichts einer aus einer Stoffsuspension hergestellten Faserstoffbahn, insbesondere Papier-, Karton- oder Tissuebahn, zeichnet sich dadurch aus, dass sowohl das Fasergewicht und der Wassergehalt als auch der Füllstoffanteil der Faserstoffbahn jeweils auf der Basis einer nicht-radiometrischen optischen Messung, insbesondere infrarottechnischen Messung, ermittelt und das Flächengewicht der Faserstoffbahn aus den auf diese Weise ermittelten Werten des Fasergewichts, des Wassergehalts und des Füllstoffanteils bestimmt wird.

Mit einer solchen Ausgestaltung des Verfahrens wird eine wesentlich genauere auf nicht-radiometrischen Messprinzipien basierende Bestimmung des Flächengewichts einer Faserstoffbahn wie insbesondere einer Papier-, Karton- oder Tissuebahn gewährleistet. Der ebenfalls auf der Basis einer nicht-radiometrischen optischen Messung ermittelte Füllstoffanteil kann somit insbesondere als Korrekturglied bei der nicht-radiometrischen Fasergewichtsmessung berücksichtigt werden.

Erfindungsgemäß wird somit nicht nur das Fasergewicht und der Wassergehalt, sondern zusätzlich auch der Füllstoffanteil der Faserstoffbahn auf der Basis einer nicht-radiometrischen optischen Messung, insbesondere infrarottechnischen Messung, ermittelt. Die jeweiligen Messungen sind damit nicht nur genauer, sie können auch schneller und insbesondere online bzw. während der Herstellung der Faserstoffbahn durchgeführt werden. Die Erfindung macht sich u.a. den Umstand zunutze, dass auch die verschiedenen Füllstoffkomponenten insbesondere im Infrarotbereich charakteristische Absorptionsbanden aufweisen. Bei den wesentlichen Bestandteilen des bei Anwendung einer nicht-radiometrischen optischen Messtechnik nunmehr miterfassten Füllstoffes handelt es sich im Allgemeinen um Kalziumcarbonat (CaCO₃), Kaolin und in seltenen Fällen Titanoxid (TiO₂).

Gemäß einer bevorzugten praktischen Ausgestaltung des erfindungsgemäßen Verfahrens erfolgen die zur Ermittlung des Fasergewichts, des Wassergehalts und des Füllstoffanteils der Faserstoffbahn durchgeführten nicht-radiometrischen optischen Messungen jeweils online bzw. während der Herstellung der Faserstoffbahn. Damit ist nicht nur eine genaue, sondern auch eine schnelle Bestimmung des Flächengewichts der Faserstoffbahn während deren Herstellung sichergestellt.

Der Füllstoffanteil der Faserstoffbahn kann auf der Basis einer nicht-radiometrischen optischen Messung des Füllstoffanteils der zur Herstellung der Faserstoffbahn verwendeten Stoffsuspension bzw. im der betreffenden Papiermaschine vorgeschalteten Konstantteil oder unmittelbar durch eine nicht-radiometrische optische Messung des Füllstoffanteils der laufenden Faserstoffbahn bzw. in der Papiermaschine ermittelt werden.

Das Fasergewicht und der Wassergehalt der Faserstoffbahn werden bevorzugt jeweils durch eine nicht-radiometrische optische Messung unmittelbar an der laufenden Faserstoffbahn ermittelt.

Gemäß einer bevorzugten praktischen Ausgestaltung des erfindungsgemäßen Verfahrens werden das Fasergewicht, der Wassergehalt und der Füllstoffanteil der Faserstoffbahn durch nicht-radiometrische optische Messungen des Fasergewichts, des Wassergehalts und des Füllstoffanteils an einer gleichen Stelle der laufenden Faserstoffbahn ermittelt.

Die betreffenden nicht-radiometrischen optischen Messungen können somit mittels einer entsprechenden Sensoreinheit an ein und derselben Stelle ermittelt werden.

Die zur Ermittlung des Fasergewichts, des Wassergehalts und/oder des Füllstoffanteils der Faserstoffbahn durchgeführte nicht-radiometrische optische Messung kann insbesondere jeweils eine Transmissions- und/oder eine Reflexionsmessung umfassen, bei der die Lichtintensität einer durch die Faserstoffbahn hindurchgetretenen bzw. einer von der Faserstoffbahn reflektierten Lichtstrahlung gemessen wird.

Dabei erfolgt die bei einer jeweiligen Transmissions- oder Reflexionsmessung durchgeführte Messung der Lichtintensität zweckmäßigerweise an für das Fasergewicht, den Wassergehalt bzw. die verschiedenen Füllstoffkomponenten charakteristischen Absorptionsbanden. Von Vorteil ist hierbei insbesondere, wenn die bei einer jeweiligen Transmissions- oder Reflexionsmessung durchgeführte Messung der Lichtintensität an für das Fasergewicht, den Wassergehalt bzw. die verschiedenen Füllstoffkomponenten charakteristischen Absorptionsbanden bei einer jeweiligen Messwellenlänge und einer jeweiligen Referenzwellenlänge erfolgt.

Dabei können beispielsweise für die Füllstoffkomponenten Kalziumcarbonat (CaCO₃) und Kaolin insbesondere Absorptionsbande bei einer Messwellenlänge im Bereich von 4,0 µm bzw. im Bereich von 2,2 µm genutzt werden, während für Zellulose und Wasser insbesondere Wellenlängen < 2 µm verwendet werden können. Die entsprechenden Referenzwellenlängen sind den jeweiligen Messwellenlängen benachbart gewählt und werden durch die betreffende Füllstoffkomponente bzw. den Zellulose- und Wasseranteil nicht beeinflusst.

Gemäß einer bevorzugten praktischen Ausgestaltung des erfindungsgemäßen Verfahrens erfolgt eine jeweilige Transmissions- oder Reflexionsmessung unter Verwendung einer Ulbricht-Kugel mit zugeordneten optischen Interferenzfiltern und Detektoren.

Bei der Ulbricht-Kugel handelt es sich um ein Bauelement der technischen Optik, das zur Erzeugung einer gleichmäßigen diffusen Strahlung aus gerichteter Strahlung dient. Zudem kann der Aufbau der jeweiligen Transmissions- bzw. Reflexionsmesseinrichtung eine breitbandige Lichtquelle wie beispielsweise eine Halogenlampe umfassen, deren Licht beispielsweise über eine Spiegelanordnung umgelenkt wird und nach einer Modulation durch eine Moduliereinrichtung wie insbesondere einen Chopper oder Chopperrad auf die Faserstoffbahn trifft. Das durch die Faserstoffbahn transmittierte bzw. von dieser reflektierte Licht kann beispielsweise über eine Linsenanordnung in die Ulbricht-Kugel gerichtet und dort gemischt werden. Die Interferenzfilter und Detektoren können insbesondere an den Wänden der Ulbricht-Kugel angebracht sein. Dabei lassen die Interferenzfilter jeweils nur Licht an der bestimmten Wellenlänge auf einen zugehörigen Detektor fallen. Das Licht zu den einzelnen Wellenlängen wird erfasst. Die Wellenlängen liegen auf den Absorptionsbanden von Wasser, Zellulose, den betreffenden Füllstoffkomponenten sowie auf zugehörigen benachbarten Referenzwellenlängen.

Es kann in speziellen Fällen auch vorgesehen sein, dass die Ulbricht-Kugel ohne zugeordnete optische Interferenzfilter und Detektoren verwendet wird.
Weiterhin kann auch vorgesehen sein, dass zwar optische Interferenzfilter und Detektoren, jedoch keine Ulbricht-Kugel verwendet werden. Auch hier lassen die Interferenzfilter jeweils nur Licht an der bestimmten Wellenlänge auf einen zugehörigen Detektor fallen.. Das Licht zu den einzelnen Wellenlängen wird erfasst. Die Wellenlängen liegen auf den Absorptionsbanden von Wasser, Zellulose, den betreffenden Füllstoffkomponenten sowie auf zugehörigen benachbarten Referenzwellenlängen.

Mit der Modulation des von der Lichtquelle ausgesandten Lichts kann eine phasensynchrone Detektion erfolgen, die zu einer Verbesserung des Signal-Rausch-Verhältnisses führt.

Das Licht der Lichtquelle wird durch die Faserstoffbahn gestreut, so dass der Gesamtlichtstrom, der auf die Ulbricht-Kugel trifft, verschiedene Richtcharakteristiken aufweist. Mittels der Ulbricht-Kugel werden diese Lichtstrahlen in eine homogene diffuse Strahlung umgewandelt. Die Leistung oder der Gesamt-Lichtstrom der Lichtstrahlen kann somit vermessen werden, ohne dass durch deren Richtcharakteristik die Messungen verfälscht werden. Zudem bietet die erzeugte diffuse Strahlung die Möglichkeit, ein fotometrisches Normal bzw. eine Referenz-Strahlungsquelle zu schaffen, um die Eigenschaften verschiedener optischer Detektoren miteinander zu vergleichen.

Eine entsprechende Verwendung einer Ulbricht-Kugel bringt insbesondere den Vorteil mit sich, dass eine gleichzeitige Messung der verschiedenen optischen Wellenlängen an einer gleichen Stelle bzw. an einem Messfleck der bewegten Faserstoffbahn möglich ist.

Gemäß einer weiteren vorteilhaften Ausgestaltung des erfindungsgemäßen Verfahrens kann eine jeweilige Transmissions- oder Reflexionsmessung auch unter Verwendung eines rotierenden, mit optischen Interferenzfiltern bestückten Filterrades erfolgen.

Auch in diesem Fall kann als Lichtquelle insbesondere wieder eine breitbandige Lichtquelle wie beispielsweise eine Halogenlampe verwendet werden. Das die Faserstoffbahn durchdringende bzw. von dieser reflektierte Licht fällt über das insbesondere mit hoher Geschwindigkeit rotierende Filterrad auf eine Detektoranordnung, deren Ausgangssignale in einer Auswerteeinrichtung ausgewertet werden. Auch im vorliegenden Fall ist wieder sowohl eine Transmissions- als auch eine Reflexionsmessung denkbar.

Wie bereits erwähnt, kann eine jeweilige Transmissions- oder Reflexionsmessung insbesondere unter Verwendung einer breitbandigen Lichtquelle erfolgen.

In bestimmten Fällen ist insbesondere auch von Vorteil, wenn eine jeweilige Transmissions- oder Reflexionsmessung unter Verwendung einer Laserlichtquelle erfolgt.

Gemäß einer weiteren bevorzugten praktischen Ausführungsform des erfindungsgemäßen Verfahrens erfolgt eine jeweilige Transmissions- oder Reflexionsmessung unter Verwendung einer eine Mehrzahl von Lasern unterschiedlicher Lichtwellenlänge umfassenden Laserlichtquelle, wobei die Lichtwellenlängen der Laser den für das Fasergewicht, den Wassergehalt bzw. die verschiedenen Füllstoffkomponenten charakteristischen Mess- und Referenzwellenlängen entsprechen.

Dabei kann das Licht der Laser auf die Faserstoffbahn gerichtet und das durch die Faserstoffbahn transmittierte bzw. von dieser reflektierte Licht dann jeweils von einem Detektor gemessen werden. Die Laserlichtstrahlen können im Bereich eines gleichen Messflecks auf die bewegte Faserstoffbahn treffen, so dass auch im vorliegenden Fall wieder eine sogenannte "SameSpot"-Eigenschaft der betreffenden Messanordnung gegeben ist. Wie bereits erwähnt, ist auch hier wieder sowohl eine Transmissions- als auch eine Reflexionsmessung möglich.

Die erfindungsgemäße Vorrichtung zur Bestimmung des Flächengewichts einer aus einer Stoffsuspension hergestellten Faserstoffbahn, insbesondere Papier-, Karton- oder Tissuebahn umfasst entsprechend eine nicht-radiometrische optische, insbesondere infrarotoptische Messeinrichtung und eine Auswerteeinrichtung zur Ermittlung des Fasergewichts, des Wassergehalts und des Füllstoffanteils der Faserstoffbahn auf der Basis einer jeweiligen mittels der nicht-radiometrischen optischen Messeinrichtung durchgeführten nicht-radiometrischen optischen Messung und zur Bestimmung des Flächengewichts der Faserstoffbahn aus den so ermittelten Werten des Fasergewichts, des Wassergehalts und des Füllstoffanteils.

Bevorzugt ist die nicht-radiometrische optische Messeinrichtung zur Durchführung von nicht-radiometrischen optischen Online-Messungen bzw. nicht-radiometrischen optischen Messungen während der Herstellung der Faserstoffbahn ausgeführt.

Von Vorteil ist insbesondere auch, wenn die nicht-radiometrische optische Messeinrichtung Mittel zur nicht-radiometrischen optischen Messung des Füllstoffanteils der zur Herstellung der Faserstoffbahn verwendeten Stoffsuspension und/oder Mittel zur nicht-radiometrischen optischen Messung des Füllstoffanteils unmittelbar an der laufenden Faserstoffbahn umfasst.

Gemäß einer weiteren vorteilhaften Ausführungsform der erfindungsgemäßen Vorrichtung umfasst die nicht-radiometrische optische Messeinrichtung Mittel zur jeweiligen nicht-radiometrischen optischen Messung des Fasergewichts und des Wassergehalts der Faserstoffbahn unmittelbar an der laufenden Faserstoffbahn.

Von Vorteil ist insbesondere auch, wenn die nicht-radiometrische optische Messeinrichtung eine Sensoreinheit zur nicht-radiometrischen optischen Messung des Fasergewichts, des Wassergehalts und des Füllstoffanteils der Faserstoffbahn an einer gleichen Stelle der laufenden Faserstoffbahn umfasst.

Die nicht-radiometrische optische Messeinrichtung kann insbesondere eine Transmissions- und/oder eine Reflexionsmesseinheit zur Messung der Lichtintensität einer durch die Faserstoffbahn hindurchgetretenen bzw. einer von der Faserstoffbahn reflektierten Lichtstrahlung umfassen.

Dabei ist die Transmissions- bzw. Reflexionsmesseinheit bevorzugt zur Messung der Lichtintensität an für das Fasergewicht, den Wassergehalt bzw. die verschiedenen Füllstoffkomponenten charakteristischen Absorptionsbanden ausgeführt. Bevorzugt ist die Transmissions- bzw. Reflexionsmesseinheit zur Messung der Lichtintensität an für das Fasergewicht, den Wassergehalt bzw. die verschiedenen Füllstoffkomponenten charakteristischen Absorptionsbanden bei einer jeweiligen Messwellenlänge und einer jeweiligen Referenzwellenlänge ausgeführt.

Gemäß einer bevorzugten praktischen Ausführungsform der erfindungsgemäßen Vorrichtung umfasst die Transmissions- bzw. Reflexionsmesseinheit eine Ulbricht-Kugel mit zugeordneten optischen Interferenzfiltern und Detektoren.
Es kann in speziellen Fällen auch vorgesehen sein, dass die Ulbricht-Kugel ohne zugeordnete optische Interferenzfilter und Detektoren verwendet wird.
Weiterhin kann auch vorgesehen sein, dass zwar optische Interferenzfilter und Detektoren, jedoch keine Ulbricht-Kugel verwendet werden. Auch hier lassen die Interferenzfilter jeweils nur Licht an der bestimmten Wellenlänge auf einen zugehörigen Detektor fallen.. Das Licht zu den einzelnen Wellenlängen wird erfasst. Die Wellenlängen liegen auf den Absorptionsbanden von Wasser, Zellulose, den betreffenden Füllstoffkomponenten sowie auf zugehörigen benachbarten Referenzwellenlängen.

Gemäß einer alternativen vorteilhaften Ausführungsform der erfindungsgemäßen Vorrichtung kann eine jeweilige Transmissions- oder Reflexionsmesseinheit auch ein rotierendes, mit optischen Interferenzfiltern bestücktes Filterrad umfassen.

Bevorzugt umfasst eine jeweilige Transmissions- oder Reflexionseinheit eine breitbandige Lichtquelle.

In bestimmten Fällen ist insbesondere auch von Vorteil, wenn eine jeweilige Transmissions- oder Reflexionseinheit eine Laserlichtquelle umfasst.

Dabei umfasst eine jeweilige Transmissions- oder Reflexionseinheit gemäß einer bevorzugten praktischen Ausführungsform der erfindungsgemäßen Vorrichtung eine eine Mehrzahl von Lasern unterschiedlicher Lichtwellenlänge aufweisende Laserlichtquelle, wobei die Lichtwellenlängen der Laser den für das Fasergewicht, den Wassergehalt bzw. die verschiedenen Füllstoffkomponenten charakteristischen Mess- und Referenzwellenlängen entsprechen.

Die Erfindung wird im Folgenden anhand von Ausführungsbeispielen unter Bezugnahme auf die Zeichnung näher erläutert; in dieser zeigen:
- Fig. 1: eine schematische Darstellung einer beispielhaften Ausführungsform einer erfindungsgemäßen Vorrichtung zur Bestimmung des Flächengewichts einer Faserstoffbahn mit einer Transmissionsmesseinheit, die eine Ulbricht-Kugel umfasst,
- Fig. 2: eine schematische Darstellung einer weiteren beispielhaften Ausführungsform einer erfindungsgemäßen Vorrichtung mit einer Transmissionsmesseinheit, die ein rotierendes, mit optischen Interferenzfiltern bestücktes Filterrad umfasst, und
- Fig. 3: eine schematische Darstellung einer weiteren beispielhaften Ausführungsform einer erfindungsgemäßen Vorrichtung mit einer Transmissionsmesseinheit, die eine Mehrzahl von Lasern unterschiedlicher Laserwellenlänge aufweisende Laserlichtquelle umfasst.

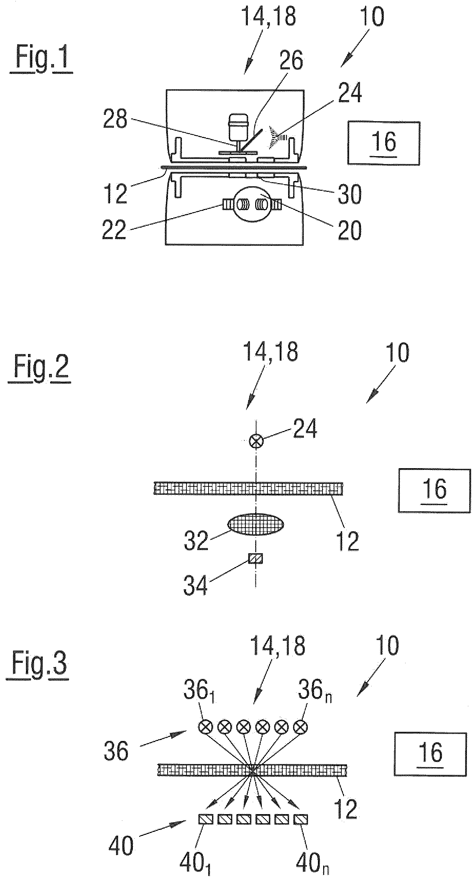

Die Fig. 1 bis 3 zeigen beispielhafte Ausführungsformen einer erfindungsgemäßen Vorrichtung 10 zur Bestimmung des Flächengewichts einer aus einer Stoffsuspension hergestellten Faserstoffbahn 12, bei der es sich insbesondere um eine Papier-, Karton- oder Tissuebahn handeln kann.

Dabei umfasst die Vorrichtung 10 jeweils eine nicht-radiometrische optische, insbesondere infrarotoptische Messeinrichtung 14 und eine Auswerteeinrichtung 16 zur Ermittlung des Fasergewichts, des Wassergehalts und des Füllstoffanteils der Faserstoffbahn 12 auf der Basis einer jeweiligen mittels der nicht-radiometrischen optischen Messeinrichtung 14 durchgeführten nicht-radiometrischen optischen Messung und zur Bestimmung des Flächengewichts der Faserstoffbahn 12 aus den so ermittelten Werten des Fasergewichts, des Wassergehalts und des Füllstoffanteils.

Die nicht-radiometrische optische Messeinrichtung 14 einer jeweiligen Vorrichtung 10 kann insbesondere zur Durchführung von nicht-radiometrischen optischen Online-Messungen bzw. nicht-radiometrischen optischen Messungen während der Herstellung der Faserstoffbahn 12 ausgeführt sein.

Die nicht-radiometrische optische Messeinrichtung 14 einer jeweiligen Vorrichtung 10 kann insbesondere Mittel zur nicht-radiometrischen optischen Messung des Füllstoffanteils der zur Herstellung der Faserstoffbahn 12 verwendeten Stoffsuspension und/oder Mittel zur nicht-radiometrischen optischen Messung des Füllstoffanteils unmittelbar an der laufenden Faserstoffbahn 12 umfassen. Der Füllstoffanteil der Faserstoffbahn kann also auf der Basis einer nicht-radiometrischen optischen Messung des Füllstoffanteils im der betreffenden Papiermaschine vorgeschalteten Konstantteil oder in der Papiermaschine unmittelbar an der laufenden Faserstoffbahn gemessen werden.

Zudem kann die nicht-radiometrische optische Messeinrichtung 14 einer jeweiligen Vorrichtung 10 insbesondere Mittel zur jeweiligen nicht-radiometrischen optischen Messung des Fasergewichts und des Wassergehalts der Faserstoffbahn 12 unmittelbar an der laufenden Faserstoffbahn 12 umfassen.

Bei den in den Fig. 1 bis 3 dargestellten verschiedenen Ausführungsformen einer erfindungsgemäßen Vorrichtung 10 umfasst die nicht-radiometrische optische Messeinrichtung 14 jeweils eine Sensoreinheit 18 zur nicht-radiometrischen optischen Messung des Fasergewichts, des Wassergehalts und des Füllstoffanteils der Faserstoffbahn 12 an einer gleichen Stelle der laufenden Faserstoffbahn 12, so dass jeweils eine sogenannte "SameSpot"-Eigenschaft der betreffenden Messanordnung gegeben ist.

Die nicht-radiometrische optische Messeinrichtung 14 bzw. Sensoreinheit 18 einer jeweiligen Vorrichtung 10 kann insbesondere eine Transmissions- und/oder eine Reflexionsmesseinheit zur Messung der Lichtintensität einer durch die Faserstoffbahn 12 hindurchgetretenen bzw. einer von der Faserstoffbahn 12 reflektierten Lichtstrahlung umfassen. Dabei kann die jeweilige Transmissions- bzw. Reflexionsmesseinheit zur Messung der Lichtintensität an für das Fasergewicht, den Wassergehalt bzw. die verschiedenen Füllstoffkomponenten charakteristischen Absorptionsbanden ausgeführt sein, wobei die jeweilige Transmissions- bzw. Reflexionsmesseinheit insbesondere zur Messung der Lichtintensität an für das Fasergewicht, den Wassergehalt bzw. die verschiedenen Füllstoffkomponenten charakteristischen Absorptionsbanden bei einer jeweiligen Messwellenlänge und einer jeweiligen Referenzwellenlänge ausgeführt sein kann.

Die wesentlichen Bestandteile des Füllstoffes sind im Allgemeinen Kalziumcarbonat (CaCO₃), Kaolin und in selteneren Fällen Titanoxid (TiO₂). Für die Füllstoffkomponente Kalziumcarbonat (CaCO₃) kann insbesondere die Absorptionsbande bei einer Messwellenlänge im Bereich von 4,0 µm und für die Füllstoffkomponente Kaolin insbesondere die Absorptionsbande bei einer Messwellenlänge im Bereich von 2,2 µm genutzt werden. Die betreffenden Messwellenlängen für Zellulose und Wasser sind insbesondere <2 µm. Wie bereits erwähnt, kann die Messung der Lichtintensität in Transmission oder in Reflexion erfolgen.

Fig. 1 zeigt in schematischer Darstellung eine beispielhafte Ausführungsform einer erfindungsgemäßen Vorrichtung 10, bei der die nicht-radiometrische optische Messeinrichtung 14 eine hier beispielsweise als Transmissionsmesseinheit ausgeführte Sensoreinheit 18 mit einer Ulbricht-Kugel 20 mit zugeordneten optischen Interferenzfiltern und Detektoren 22 umfasst.

Der Aufbau der Transmissionsmesseinheit bzw. Sensoreinheit 18 umfasst eine breitbandige Lichtquelle 24 wie beispielsweise eine Halogenlampe, deren Licht durch eine Spiegelanordnung 26 umgelenkt wird und nach einer Modulation durch eine Moduliereinrichtung 28 wie beispielsweise einen Chopper oder ein Chopperrad auf die Faserstoffbahn 12 trifft. Das durch die Faserstoffbahn 12 transmittierte Licht wird über eine Linsenanordnung 30 in die Ulbricht-Kugel 20 gelenkt und dort gemischt. Die Interferenzfilter und Detektoren 22 sind an den Wänden der Ulbricht-Kugel 20 angebracht. Die Interferenzfilter lassen jeweils nur Licht einer bestimmten Wellenlänge auf einen zugehörigen Detektor fallen. Das Licht zu den einzelnen Wellenlängen wird erfasst. Die Wellenlängen liegen auf den Absorptionsbanden von Wasser, Zellulose und den jeweiligen Füllstoffkomponenten sowie auf zugehörigen benachbarten Referenzwellenlängen. Ein wesentlicher Vorteil dieser Ausführungsform liegt in der gleichzeitigen Messung der optischen Wellenlängen im Bereich eines Papiermessflecks bei bewegter Faserstoffbahn, womit die bereits erwähnte "SameSpot"-Eigenschaft der Messanordnung gegeben ist.

Die Sensoreinheit 18 kann grundsätzlich auch als Reflexionsmesseinheit ausgeführt sein.

Fig. 2 zeigt in schematischer Darstellung eine alternative beispielhafte Ausführungsform einer erfindungsgemäßen Vorrichtung 10, deren hier beispielsweise als Transmissionsmesseinheit ausgeführte nicht-radiometrische optische Messeinrichtung 14 bzw. Sensoreinheit 18 ein Filterrad 32 umfasst, das mit optischen Interferenzfiltern bestückt ist. Das Filterrad 32 rotiert mit hoher Geschwindigkeit. Als Lichtquelle ist wieder eine breitbandige Lichtquelle 24 wie beispielsweise eine Halogenlampe vorgesehen.

Das Licht der Lichtquelle 24 durchdringt die Faserstoffbahn 12 und fällt dann über das rotierende Filterrad 32 auf einen Detektor 34. Dabei werden die interessierenden zu messenden wellenlängenspezifischen Signale über den Detektor 34 sequentiell bzw. zeitlich nacheinander erfasst und zur Auswertung wieder einer Auswerteeinrichtung 16 zugeführt.

Auch im vorliegenden Fall kann die Sensoreinheit 18 anstatt als Transmissionsmesseinheit auch wieder als Reflexionsmesseinheit ausgeführt sein.

Die jeweilige Transmissions- oder Reflexionsmesseinheit kann insbesondere auch eine Laserlichtquelle umfassen.

Fig. 3 zeigt in schematischer Darstellung eine weitere beispielhafte Ausführungsform einer erfindungsgemäßen Vorrichtung 10, bei der die hier beispielsweise als Transmissionsmesseinheit ausgeführte nicht-radiometrische optische Messeinrichtung 14 bzw. Sensoreinheit 18 eine eine Mehrzahl von Lasern 36₁ bis 36ₙ unterschiedlicher Laserwellenlänge aufweisende Laserlichtquelle 36 umfasst. Dabei entsprechen die Lichtwellenlängen der verschiedenen Laser 36₁ bis 36ₙ der Laserlichtquelle 36 den Mess- und Referenzwellenlängen von Wasser, Zellulose und den betreffenden Füllstoffkomponenten wie beispielsweise Kalziumcarbonat (CaCO₃) und Kaolin. Das Licht der Laser 36₁ bis 36ₙ wird auf die Faserstoffbahn 12 gerichtet. Anschließend wird das durch die Faserstoffbahn 12 transmittierte Licht jeweils von einem Detektor 40₁ bis 40ₙ einer Detektoranordnung 40 erfasst. Die Laserlichtstrahlen der verschiedenen Laser 36₁ bis 36ₙ treffen an demselben Messfleck auf die Faserstoffbahn 12, so dass auch im vorliegenden Fall wieder eine sogenannte "SameSpot"-Eigenschaft der Messanordnung gegeben ist.

Ebenso wie bei den anhand der Fig. 1 und 2 beschriebenen Ausführungsbeispielen kann die Sensoreinheit 18 auch im vorliegenden Fall anstelle einer Transmissionsmesseinheit wieder eine Reflexionsmesseinheit umfassen.

In den verschiedenen Figuren sind einander entsprechenden Teilen gleiche Bezugszeichen zugeordnet.

Aufgrund der erfindungsgemäßen Ausbildung ist eine genauere, auf nicht-radiometrischen Messprinzipien basierende Bestimmung des Flächengewichts einer Faserstoffbahn wie insbesondere einer Papier-, Karton- oder Tissuebahn gewährleistet. Dabei kann der im Konstantteil oder in der Papiermaschine gemessene Aschegehalt oder Füllstoffanteil insbesondere als Korrekturglied bei der nicht-radiometrischen Fasergewichtsmessung herangezogen werden. Dies ist insbesondere bei der Herstellung von Tissuepapier von Vorteil, nachdem das Flächengewicht bei der Herstellung von Tissuepapier infolge der starken Schwankungen des Füllstoffanteils relativ stark schwanken und in einem großen Bereich zwischen 8 und 60 g/m² variieren kann.

### Bezugszeichenliste

- 10: Vorrichtung zur Bestimmung des Flächengewichts
- 12: Faserstoffbahn
- 14: nicht-radiometrische optische Messeinrichtung
- 16: Auswerteeinrichtung
- 18: Sensoreinheit
- 20: Ulbricht-Kugel
- 22: Interferenzfilter, Detektor
- 24: breitbandige Lichtquelle
- 26: Spiegelanordnung
- 28: Moduliereinrichtung
- 30: Linsenanordnung
- 32: Filterrad
- 34: Detektor
- 36: Laserlichtquelle
- 36₁ - 36ₙ: Laser
- 40: Detektoranordnung
- 40₁ - 40ₙ: Detektor

## Patentansprüche

1. Verfahren zur Bestimmung des Flächengewichts einer aus einer Stoffsuspension hergestellten Faserstoffbahn (12), insbesondere Papier-, Karton- oder Tissuebahn, bei dem sowohl das Fasergewicht und der Wassergehalt als auch der Füllstoffanteil der Faserstoffbahn (12) jeweils auf der Basis einer nicht-radiometrischen optischen Messung, insbesondere infrarottechnischen Messung, ermittelt und das Flächengewicht der Faserstoffbahn (12) aus den auf diese Weise ermittelten Werten des Fasergewichts, des Wassergehalts und des Füllstoffanteils bestimmt wird, wobei die zur Ermittlung des Fasergewichts, des Wassergehalts und des Füllstoffanteils der Faserstoffbahn (12) durchgeführte nicht-radiometrische optische Messung jeweils eine Transmissions- und/oder eine Reflexionsmessung umfasst, bei der die Lichtintensität einer durch die Faserstoffbahn (12) hindurchgetretenen bzw. einer von der Faserstoffbahn (12) reflektierten Lichtstrahlung gemessen wird, wobei diese Messung an für das Fasergewicht, den Wassergehalt bzw. die verschiedenen Füllstoffkomponenten charakteristischen Absorptionsbanden bei einer jeweiligen Messwellenlänge und einer jeweiligen Referenzwellenlänge erfolgt.

2. Verfahren nach Anspruch 1,
**dadurch gekennzeichnet, dass** die zur Ermittlung des Fasergewichts, des Wassergehalts und des Füllstoffanteils der Faserstoffbahn (12) durchgeführten nicht-radiometrischen optischen Messungen jeweils online bzw. während der Herstellung der Faserstoffbahn (12) erfolgen.

3. Verfahren nach Anspruch 1 oder 2,
**dadurch gekennzeichnet, dass** der Füllstoffanteil der Faserstoffbahn (12) auf der Basis einer nicht-radiometrischen optischen Messung des Füllstoffanteils der zur Herstellung der Faserstoffbahn (12) verwendeten Stoffsuspension oder unmittelbar durch eine nicht-radiometrische optische Messung des Füllstoffanteils der laufenden Faserstoffbahn (12) ermittelt wird.

4. Verfahren nach zumindest einem der vorstehenden Ansprüche,
**dadurch gekennzeichnet, dass** eine jeweilige Transmissions- oder Reflexionsmessung unter Verwendung einer Ulbricht-Kugel (20) und/oder optischen Interferenzfiltern und Detektoren (22) erfolgt.

5. Verfahren nach zumindest einem der vorstehenden Ansprüche,
**dadurch gekennzeichnet, dass** eine jeweilige Transmissions- oder Reflexionsmessung unter Verwendung eines rotierenden, mit optischen Interferenzfiltern bestückten Filterrades (32) erfolgt.

6. Verfahren nach zumindest einem der vorstehenden Ansprüche,
**dadurch gekennzeichnet, dass** eine jeweilige Transmissions- oder Reflexionsmessung unter Verwendung einer breitbandigen Lichtquelle (24) oder zumindest einer Laserlichtquelle (36) erfolgt.

7. Vorrichtung (10) zur Bestimmung des Flächengewichts einer aus einer Stoffsuspension hergestellten Faserstoffbahn (12), insbesondere Papier-, Karton- oder Tissuebahn, insbesondere zur Durchführung des Verfahrens nach zumindest einem der vorstehenden Ansprüche, mit einer nicht-radiometrischen optischen, insbesondere infrarotoptischen Messeinrichtung (14) und einer Auswerteeinrichtung (16) zur Ermittlung des Fasergewichts, des Wassergehalts und des Füllstoffanteils der Faserstoffbahn (12) auf der Basis einer jeweiligen mittels der nicht-radiometrischen optischen Messeinrichtung (14) durchgeführten nicht-radiometrischen optischen Messung und zur Bestimmung des Flächengewicht der Faserstoffbahn (12) aus den so ermittelten Werten des Fasergewichts, des Wassergehalts und des Füllstoffanteils, wobei die nicht-radiometrische optische Messeinrichtung (14) eine Transmissions- und/oder eine Reflexionsmesseinheit zur Messung der Lichtintensität einer durch die Faserstoffbahn (12) hindurchgetretenen bzw. einer von der Faserstoffbahn (12) reflektierten Lichtstrahlung umfasst, wobei die nicht-radiometrische optische Messeinrichtung (14) zur Messung der Lichtintensität an für das Fasergewicht, den Wassergehalt bzw. die verschiedenen Füllstoffkomponenten charakteristischen Absorptionsbanden bei einer jeweiligen Messwellenlänge und einer jeweiligen Referenzwellenlänge ausgeführt ist.

8. Vorrichtung nach Anspruch 7,
**dadurch gekennzeichnet, dass** die nicht-radiometrische optische Messeinrichtung (14) Mittel zur nicht-radiometrischen optischen Messung des Füllstoffanteils der zur Herstellung der Faserstoffbahn (12) verwendeten Stoffsuspension und/oder Mittel zur nicht-radiometrischen optischen Messung des Füllstoffanteils unmittelbar an der laufenden Faserstoffbahn (12) umfasst.

9. Vorrichtung nach einem der Ansprüche 7 bis 8,
**dadurch gekennzeichnet, dass** die Transmissions- bzw. Reflexionsmesseinheit eine Ulbricht-Kugel (20) und/oder optische Interferenzfilter und Detektoren (22) umfasst.

10. Vorrichtung nach einem der Ansprüche 7 bis 9,
**dadurch gekennzeichnet, dass** die Transmissions- oder Reflexionsmesseinheit eine breitbandige Lichtquelle (24) oder zumindest eine Laserlichtquelle (36) umfasst.

## Claims

1. Method for establishing the weight per unit area of a fibrous web (12) produced from a stock suspension, in particular a paper, cardboard or tissue web, in which not only the fibre weight and the water content but also the filler content of the fibrous web (12) are determined in each case on the basis of a non-radiometric optical measurement, in particular a measurement using infrared technology, and the weight per unit area of the fibrous web (12) is established from the values determined in this way for the fibre weight, the water content and the filler content, wherein the non-radiometric optical measurement carried out for determining the fibre weight, the water content and the filler content of the fibrous web (12) in each case comprises a transmission and/or reflection measurement, in which the light intensity of a luminous radiation passing through the fibrous web (12) or reflected by the fibrous web (12) is measured, wherein this measurement is performed at absorption bands characteristic of the fibre weight, the water content and the various filler components at a respective measuring wavelength and a respective reference wavelength.

2. Method according to Claim 1,
**characterized in that** the non-radiometric optical measurements carried out for determining the fibre weight, the water content and the filler content of the fibrous web (12) are in each case performed online or during the production of the fibrous web (12).

3. Method according to Claim 1 or 2,
**characterized in that** the filler content of the fibrous web (12) is determined on the basis of a non-radiometric optical measurement of the filler content of the stock suspension used for producing the fibrous web (12) or directly by a non-radiometric optical measurement of the filler content of the running fibrous web (12).

4. Method according to at least one of the preceding claims,
**characterized in that** a respective transmission or reflection measurement is performed by using an Ulbricht sphere (20) and/or optical interference filters and detectors (22).

5. Method according to at least one of the preceding claims,
**characterized in that** a respective transmission or reflection measurement is performed by using a rotating filter wheel (32) fitted with optical interference filters.

6. Method according to at least one of the preceding claims,
**characterized in that** a respective transmission or reflection measurement is performed by using a broadband light source (24) or at least one laser light source (36).

7. Apparatus (10) for establishing the weight per unit area of a fibrous web (12) produced from a stock suspension, in particular a paper, cardboard or tissue web, in particular for carrying out the method according to at least one of the preceding claims, with a non-radiometric optical, in particular infrared-optical, measuring device (14) and an evaluating device (16) for determining the fibre weight, the water content and the filler content of the fibrous web (12) on the basis of a respective non-radiometric optical measurement carried out by means of the non-radiometric optical measuring device (14) and for establishing the weight per unit area of the fibrous web (12) from the values determined in this way for the fibre weight, the water content and the filler content, wherein the non-radiometric optical measuring device (14) comprises a transmission and/or reflection measuring unit for measuring the light intensity of a luminous radiation passing through the fibrous web (12) or reflected by the fibrous web (12), wherein the non-radiometric optical measuring device (14) is designed for measuring the light intensity at absorption bands characteristic of the fibre weight, the water content and the various filler components at a respective measuring wavelength and a respective reference wavelength.

8. Apparatus according to Claim 7,
**characterized in that** the non-radiometric optical measuring device (14) comprises means for the non-radiometric optical measurement of the filler content of the stock suspension used for producing the fibrous web (12) and/or means for the non-radiometric optical measurement of the filler content directly on the running fibrous web (12).

9. Apparatus according to either of Claims 7 and 8,
**characterized in that** the transmission or reflection measuring unit comprises an Ulbricht sphere (20) and/or optical interference filters and detectors (22).

10. Apparatus according to one of Claims 7 to 9,
**characterized in that** the transmission or reflection measuring unit comprises a broadband light source (24) or at least one laser light source (36).

## Revendications

1. Procédé de détermination de la masse surfacique d'une bande de matière fibreuse (12) fabriquée à partir d'une suspension de matière, notamment d'une bande de papier, de carton ou de papier absorbant, dans lequel on détermine tant le poids des fibres et la teneur en eau que la fraction de matière de charge de la bande de matière fibreuse (12) respectivement sur la base d'une mesure optique non radiométrique, notamment d'une mesure par infrarouge, et on détermine la masse surfacique de la bande de matière fibreuse (12) à partir des valeurs ainsi déterminées du poids des fibres, de la teneur en eau et de la fraction de matière de charge,
dans lequel la mesure optique non radiométrique effectuée pour déterminer le poids des fibres, la teneur en eau et la fraction de matière de charge de la bande de matière fibreuse (12) comprend respectivement une mesure de transmission et/ou une mesure de réflexion, lors de laquelle on mesure l'intensité lumineuse d'un rayonnement lumineux ayant traversé la bande de matière fibreuse (12) ou d'un rayonnement lumineux réfléchi par la bande de matière fibreuse (12), dans lequel ladite mesure est effectuée sur des bandes d'absorption caractéristiques du poids des fibres, de la teneur en eau ou des différents constituants de la matière de charge, à une longueur d'onde de mesure respective et à une longueur d'onde de référence respective.

2. Procédé selon la revendication 1,
**caractérisé en ce que** les mesures optiques non radiométriques réalisées pour déterminer le poids des fibres, la teneur en eau et la fraction de matière de charge de la bande de matière fibreuse (12) sont respectivement effectuées en ligne ou durant la fabrication de la bande de matière fibreuse (12).

3. Procédé selon la revendication 1 ou 2,
**caractérisé en ce que** la fraction de matière de charge de la bande de matière fibreuse (12) est déterminée sur la base d'une mesure optique non radiométrique de la fraction de matière de charge de la suspension de matière utilisée pour fabriquer la bande de matière fibreuse (12) ou directement par une mesure optique non radiométrique de la fraction de matière de charge de la bande de matière fibreuse (12) en mouvement.

4. Procédé selon au moins l'une des revendications précédentes, **caractérisé en ce qu'**une mesure de transmission ou de réflexion respective est effectuée en utilisant une sphère d'intégration (20) et/ou des filtres d'interférence optiques et des détecteurs (22).

5. Procédé selon au moins l'une des revendications précédentes, **caractérisé en ce qu'**une mesure de transmission ou de réflexion respective est effectuée en utilisant une roue de filtres tournante (32) équipée de filtres d'interférence optiques.

6. Procédé selon au moins l'une des revendications précédentes, **caractérisé en ce qu'**une mesure de transmission ou de réflexion respective est effectuée en utilisant une source lumineuse à large bande (24) ou au moins une source lumineuse à laser (36).

7. Dispositif (10) de détermination de la masse surfacique d'une bande de matière fibreuse (12) fabriquée à partir d'une suspension de matière, notamment de papier, de carton ou de papier absorbant, notamment pour la mise en oeuvre du procédé selon au moins l'une des revendications précédentes, comportant un dispositif de mesure optique non radiométrique, notamment optique infrarouge (14), et un dispositif d'évaluation (16) destiné à déterminer le poids des fibres, la teneur en eau et la fraction de matière de charge de la bande de matière fibreuse (12) sur la base d'une mesure optique non radiométrique respectivement réalisée au moyen du dispositif optique non radiométrique (14) et à déterminer la fraction de matière fibreuse (12) à partir des valeurs ainsi déterminées du poids des fibres, de la teneur en eau et de la fraction de matière de charge, dans lequel le dispositif de mesure optique non radiométrique (14) comprend une unité de mesure de transmission et/ou de réflexion destinée à mesurer l'intensité lumineuse d'un rayonnement lumineux ayant traversé la bande de matière fibreuse (12) ou réfléchi par la bande de matière fibreuse (12), dans lequel le dispositif de mesure optique non radiométrique (14) est conçu pour mesurer l'intensité lumineuse de bandes d'absorption caractéristiques du poids des fibres, de la teneur en eau ou des différents constituants de la matière de charge, à une longueur d'onde de mesure respective et à une longueur d'onde de référence respective.

8. Dispositif selon la revendication 7,
**caractérisé en ce que** le dispositif de mesure optique non radiométrique (14) comprend des moyens de mesure optique non radiométrique de la fraction de matière de charge de la suspension de matière utilisée pour fabriquer la bande de matière fibreuse (12) et/ou des moyens de mesure optique non radiométrique de la fraction de matière de charge, directement sur la bande de matière fibreuse (12) en mouvement.

9. Dispositif selon l'une quelconque des revendications 7 à 8,
**caractérisé en ce que** l'unité de mesure de transmission ou de réflexion comprend une sphère d'intégration (20) et/ou des filtres d'interférence et des détecteurs optiques (22).

10. Dispositif selon l'une quelconque des revendications 7 à 9,
**caractérisé en ce que** l'unité de mesure de transmission ou de réflexion comprend une source lumineuse à large bande (24) ou au moins une source lumineuse à laser (36).
